# EUROPÄISCHE PATENTANMELDUNG

(11) **EP 1 038 969 A2**
(43) Veröffentlichungstag der Anmeldung: **27.09.2000**
(21) Anmeldenummer: 00104113.6
(22) Anmeldetag: 29.02.2000
(51) Int. Cl.: C12P 13/04

(54) **Verfahren zur fermentativen Herstellung von L-Aminosäuren unter Verwendung coryneformer Bakterien**

(30) Priorität: 19.03.1999 DE 19912384
(71) Anmelder: Degussa-Hüls Aktiengesellschaft, 60287 Frankfurt am Main (DE)
(72) Erfinder: Molenaar, Douwe, Dr., 52499 Baesweiler (DE); Van der Rest, Michel Eduard, Dr., 5913 Venlo (NL); Möckel, Bettina, Dr., 33602 Bielefeld (DE)

(57) **Zusammenfassung**

Die Erfindung betrifft einVerfahren zur fermentativen Herstellung von L-Aminosäuren unter Verwendung coryneformer Bakterien, in denen das mqo-Gen verstärkt wird.

## Beschreibung

Gegenstand der Erfindung ist ein Verfahren zur fermentativen Herstellung von L-Aminosäuren, insbesondere L-Lysin unter Verwendung von coryneformen Bakterien, in denen das mqo-Gen verstärkt wird.

### Stand der Technik

L-Aminosäuren, insbesondere L-Lysin finden in der Tierernährung, in der Humanmedizin und in der pharmazeutischen Industrie Anwendung.

Es ist bekannt, daß diese Aminosäuren durch Fermentation von Stämmen coryneformer Bakterien insbesondere Corynebacterium glutamicum hergestellt werden. Wegen der großen Bedeutung wird ständig an der Verbesserung der Hersteilverfahren gearbeitet. Verfahrensbesserungen können fermentationstechnische Maßnahmen wie z.B. Rührung und Versorgung mit Sauerstoff, oder die Zusammensetzung der Nährmedien wie z.B. die Zuckerkonzentration während der Fermentation, oder die Aufarbeitung zur Produktform durch z.B. Ionenaustauschchromatographie oder die intrinsischen Leistungseigenschaften des Mikrorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Lysin-Analogon S-(2-Aminoethyl)-Cystein oder auxotroph für regulatorisch bedeutsame Aminosäuren sind und L-Aminosäuren produzieren.

Seit einigen Jahren werden ebenfalls Methoden der rekombinanten DNA-Technik zur Stammverbesserung L-Aminosäure produzierender Stämme von Corynebacterium glutamicum eingesetzt, indem man einzelne Aminosäure-Biosynthesegene amplifiziert und die Auswirkung auf die L-Aminosäure-Produktion untersucht. Übersichtsartikel hierzu findet man unter anderem bei Kinoshita ("Glutamic Acid Bacteria", in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6, 261-272 (1994)), Jetten und Sinskey (Critical Reviews in Biotechnology 15, 73-103 (1995)) und Sahm et al. (Annuals of the New York Academy of Science 782, 25-39 (1996)).

### Aufgabe der Erfindung

Die Erfinder haben sich zur Aufgabe gestellt, neue Maßnahmen zur verbesserten fermentativen Herstellung von L-Aminosäuren, insbesondere L-Lysin bereitzustellen.

### Beschreibung der Erfindung

L-Aminosäuren, insbesondere L-Lysin finden in der Tierernährung, in der Humanmedizin und in der pharmazeutischen Industrie Anwendung. Es besteht daher ein allgemeines Interesse daran neue, verbesserte Verfahren zur Herstellung dieser Verbindungen bereitzustellen.

Wenn im folgenden L-Lysin oder Lysin erwähnt wird, ist damit nicht nur die Base sondern es sind auch die Salze wie z. B. Lysin-Monohydrochlorid oder Lysin-Sulfat gemeint.

Gegenstand der Erfindung ist ein Verfahren zur fermentativen Herstellung von L-Aminosäuren, insbesondere L-Lysin unter Verwendung von coryneformen Bakterien, die insbesondere bereits die gewünschte Aminosäure produzieren und in denen die für das Enzym Malat:Chinon Oxidoreduktase codierende Nucleotidsequenz (mqo-Gen) verstärkt, insbesondere überexprimiert wird.

Bevorzugte Ausführungsformen finden sich in den Ansprüchen.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können L-Aminosäuren, insbesondere L-Lysin aus Glucose, Saccharose, Lactose, Fructose, Maltose, Melasse, Stärke, Cellulose oder aus Glycerin und Ethanol herstellen. Es handelt sich um Vertreter coryneformer Bakterien, insbesondere der Gattung Corynebacterium. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind die bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Aminosäuren, insbesondere L-Lysin produzierende Mutanten bzw. Stämme, wie beispielsweise
Corynebacterium glutamicum FERM-P 1709
Brevibacterium flavum FERM-P 1708
Brevibacterium lactofermentum FERM-P 1712
Brevibacterium flavum FERM-P 6463 und
Brevibacterium flavum FERM-P 6464.

Die Erfinder fanden heraus, daß coryneforme Bakterien nach Überexpression der Malat:Chinon Oxidoreduktase in verbesserter Weise L-Aminosäuren, insbesondere L-Lysin produzieren.

Das mqo-Gen kodiert für das Enzym Malat:Chinon Oxidoreduktase (EC 1.1.99.16), welches die Oxidation von Malat zu Oxalacetat unter Weitergabe der Elektronen an Ubichinon-1 katalysiert (Molenaar et al., European Journal of Biochemistry 254, 395 - 403 (1998)). Die Nukleotidsequenz des mqo-Gens von Corynebacterium glutamicum wurde ebenfalls von Molenaar et al. (European Journal of Biochemistry 254, 395 - 403 (1998)) bestimmt und ist bei der Nukleotidsequenz Datenbank des National Center for Biotechnology Information (NCBI, Behesda, MD, USA) unter der Accession Number AJ 22 4946 allgemein verfügbar.

Das von Molenaar et al. (European Journal of Biochemistry 254,395-403 (1998)) beschriebene mqo-Gen von C. glutamicum kann erfindungsgemäß verwendet werden. Weiterhin können Allele des mqo-Gens verwendet werden, die sich aus der Degeneriertheit des genetischen Codes oder durch funktionsneutrale Sinnmutationen (sense mutations) ergeben.

Zur Erzielung einer Überexpression kann die Kopienzahl der entsprechenden Gene erhöht werden, oder es kann die Promotor- und Regulationsregion, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich die Expression im Verlaufe der fermentativen L-Lysin-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EP-B 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)) bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Ein Beispiel für ein Plasmid mit dessen Hilfe die Malat:Chinon Oxidoreduktase überexprimiert werden kann ist pRM17 (Molenaar et al., 1998, European Journal of Biochemistry 254, 395 - 403). Plasmid pRM17 beruht auf dem Pendelvektor pJC1, der bei Cremer et al. (Molecular and General Genetics 220, 478 - 480) beschrieben ist.

Zusätzlich kann es für die Produktion der L-Aminosäuren vorteilhaft sein neben der Malat:Chinon Oxidoreduktase ein oder mehrere Enzyme des entsprechenden Biosyntheseweges zu überexprimieren. So kann beispielsweise bei der Herstellung von L-Lysin
- gleichzeitig das für die Dihydrodipicolinat-Synthase kodierende dapA-Gen überexprimiert werden (EP-B 0 197 335), oder
- gleichzeitig ein S-(2-Aminoethyl)-Cystein - Resistenz vermittelndes DNA-Fragment amplifiziert werden (EP-A 0 088 166).

Weiterhin kann es für die Produktion von L-Aminosäuren vorteilhaft sein, neben der Überexpression der Malat:Chinon Oxidoreduktase unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedlum muß in geeigneter Weise den Ansprüchen der jeweiligen Stämme genügen. Beschreibungen von Kulturmedien verschiedener Mikroorganismen sind im Handbuch "Manual of Methods tor General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoff enthaltende Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt bis sich ein Maximum der gewünschten L-Aminosäure gebildet hat. Dieses Ziel wird normalerweise innerhalb von 10 Stunden bis 160 Stunden erreicht.

Die Analyse von L-Aminosäuren kann durch Anionenaustauschchromatographie mit anschließender Ninhydrin Derivatisierung erfolgen, so wie bei Spackman et al. (Analytical Chemistry, 30, (1958), 1190) beschrieben.

Das Corynebacterium glutamicum Stamm DM22/pRM17 wurde unter der Nummer DSM12711 bei der Deutschen Sammlung von Mikroorganismen und Zellkulturen (Braunschweig, Deutschland) nach dem Budapester Vertrag hinterlegt.

Das erfindungsgemäße Verfahren dient zur fermentativen Herstellung von L-Aminosäuren insbesondere L-Asparaginsäure, L-Asparagin, L-Homoserin, L-Threonin, L-Isoleucin und L-Methionin mit coryneformen Bakterien, insbesondere der Herstellung von L-Lysin.

### Beispiele

Die vorliegende Erfindung wird im folgenden anhand von Ausführungsbeispielen näher erläutert.

Zu diesem Zweck wurden Versuche mit dem L-Lysin produzierenden Corynebacterium glutamicum Stamm DSM5715, (EP-B- 0 435 132) durchgeführt, in denen die Vorteilhaftigkeit des beanspruchten Verfahrens deutlich wird:

### Beispiel 1

### Herstellung von L-Lysin-Produzenten mit verstärkter Malat:Chinon Oxidoreduktase

Der Stamm DSM5715, wurde wie bei Liebl et al. (FEMS Microbiology Letters 65, 299-304 (1989)) mit dem Plasmid pRM17 (Molenaar et al., 1998, European Journal of Biochemistry 254 (395 - 403)) transformiert. Die Selektion der Transformanten erfolgte auf LBHIS-Agar, der mit 25 mg/l Kanamycin supplementiert worden war.LBHIS-Agar besteht aus LB Medium(Sambrook et al. (Molecular Cloning a Laboratory Manual (1989) Cold Spring Harbour Laboratories)), das mit 37 g/l Hirn Herz Bouillon der Firma Merck (Darmstadt, Deutschland), 0,5 M Sorbitol und 15 g/l Agar-Agar supplementiert wurde. Auf diese Weise entstand der Stamm DSM5715/pRM17. Der Stamm DSM5715/pJC1 wurde auf die gleiche Weise hergestellt.

### Beispiel 2

### Herstellung von L-Lysin

Die Stämme DSM5715/pRM17 und DSM5715/pJC1 wurden zunächst auf Hirn-Herz Agar, der mit Kanamycin (25 mg/l) supplementiert worden war, für 24 Stunden bei 33°C inkubiert. Für die Kultivierung in Flüssigmedium wurde Medium CgIII (Kase & Nakayama, Agricultural and Biological Chemistry 36 (9) 1611- 1621 (1972)) verwendet, das zusätzlich mit Kanamycin (25 mg/l) supplementiert worden war. Hierzu wurden 10 ml Medium, die in 100 ml Erlenmeyerkolben mit 4 Schikanen enthalten waren, mit einer Impföse des Stammes angeimpft und die Kultur für 16 Stunden bei 240 rpm und 30°C bebrütet. Diese Kultur wurde anschließend als Vorkultur weiterverwendet.

Als Produktions- bzw. Testmedium wurde das Medium MM verwendet, das zusätzlich mit Kanamycin (25 mg/l) supplementiert worden war.

Bei dem Verfahren mit Stamm DSM5715 enthielten die entsprechenden Medien kein Kanamycin.

Die Zusammensetzung und Herstellung des Mediums MM war wie folgt:

| | |
|---|---|
| Corn Steep Liquor (CSL) | 5 g/l |
| 3-Morpholino-Propansulfonsäure (MOPS) | 20 g/l |
| Glucose | 50 g/l (getrennt autoklaviert) |

| Salze: | |
|---|---|
| (NH4)2SO4) | 25 g/l |
| KH2PO4 | 0,1 g/l |
| MgSO4* 7 H2O | 1,0 g/l |
| CaCl2*2H2O | 10 mg/l |
| FeSO4 * 7H2O | 10 mg/l |
| MnSO4*H2O | 5,0mg/l |
| Biotin | 0,3 mg/l (sterilfiltriert) |
| Thiamin*HCl | 0,2 mg/l (sterilfiltriert) |
| CaCO3 | 25 g/l |
| Leucin | 0,1 g/l |

CSL, MOPS und die Salzlösung wurden mit Ammoniakwasser auf pH 7 eingestellt und autoklaviert. Anschließend wurden die sterilen Substrat und Vitaminlösungen sowie das trocken autoklavierte CaCO3 zugesetzt.

Die Kultivierung erfolgte in 100 ml Erlenmeyerkolben mit Schikanen, die mit 10 ml des oben beschrieben Produktionsmediums beschickt worden waren. Die Kulturen wurden so mit der Vorkultur angeimpft, daß die optische Dichte beim Start 0,1 betrug. Die Kultivierung erfolgte bei 33°C und 80% Luftfeuchte.

Nach 72 Stunden Inkubation wurde die optische Dichte der Kultursuspension und die Konzentration an gebildetem L-Lysin bestimmt. Die optische Dichte wurde mit einem LP2W-Photometer der Firma Dr. Lange (Berlin, Deutschland) bei einer Messwellenlänge von 660 nm bestimmt L-Lysin wurde mit einem Aminosäureanalysator der Firma Eppendorf-BioTronik (Hamburg, Deutschland) durch Ionenaustauschchromatographie und Nachsäulenreaktion mit Ninhydrindetektion bestimmt. In Tabelle 1 ist das Ergebnis des Versuches dargestellt.

**Tabelle 1**

| Stamm | OD | L-Lysin g/l |
|---|---|---|
| DSM5715 | 10,1 | 16,4 |
| DSM5715/pJC1 | 9,9 | 16,5 |
| DSM5715/pRM17 | 10,2 | 17,8 |

### Beispiel 3

### Herstellung von Threoninproduzenten mit verstärkter Malat:Chinon Oxidoreduktase

Das Plasmid pRM17 (Molenaar et al., 1998, European Journal of Biochemistry 254 (395 - 403)) wurde nach der Elektroporationsmethode von Tauch et al.(FEMS Microbiological Letters, 123:343-347 (1994)) in Corynebacterium glutamicum DSM 5399 elektroporiert. Bei dem Stamm DSM 5399 handelt es sich um einen Threonin-Produzenten, der in der EP-B-0358940 beschrieben ist. Die Selektion von Transformanten erfolgte durch Ausplattieren des Elektroporationsansatzes auf LB Agar (Sambrook et al., Molecular cloning: a laboratory manual. 2^{nd} Ed. Cold Spring Harbor Laboratory Press, Cold Spring Harbor, N.Y., 1989), der mit 25 mg/l Kanamycin supplementiert worden war. Auf diese Weise entstand der Stamm DSM5399/pRM17.

### Beispiel 4

### Herstellung von Threonin

Der in Beispiel 3 erhaltene C. glutamicum Stamm DSM5399/pRM17 wurde in einem zur Produktion von Threonin geeigneten Nährmedium kultiviert und der Threoningehalt im Kulturüberstand bestimmt.

Dazu wurde der Stamm zunächst auf Agarplatte mit dem entsprechenden Antibiotikum (Hirn-Herz Agar mit Kanamycin (25 mg/l) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII (Rase & Nakayama, Agricultural and Biological Chemistry 36 (9) 1611- 1621 (1972)) verwendet. Diesem wurde Kanamycin (25 mg/l) zugesetzt. Die Vorkultur wurde 24 Stunden bei 33°C bei 240 rpm auf dem Schüttler inkubiert. Von dieser Vorkultur wurde eine Hauptkultur angeimpft, so daß die Anfangs-OD (660nm) der Hauptkultur 0,1 betrug. Für die Hauptkultur wurde das Medium MM-Threonin verwendet.

| Medium MM-Threonin: | |
|---|---|
| CSL (Corn Steep Liquor) | 5 g/l |
| MOPS (Morpholinopropansulfonsäure) | 20 g/l |
| Glucose(getrennt autoklaviert) | 50g/l |

| Salze: | |
|---|---|
| (NH₄)₂SO₄ | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| CaCO₃ | 25 g/l |

CSL, MOPS und die Salzlösung wurden mit Ammoniakwasser auf pH 7 eingestellt und autoklaviert. Anschließend wurden die sterilen Substrat- und Vitaminlösungen zugesetzt, sowie das trocken autoklavierte CaCO₃.

Die Kultivierung erfolgt in 10 ml Volumen in einem 100 ml Erlenmeyerkolben mit Schikanen. Es wurde Kanamycin (25 mg/l) zugesetzt. Die Kultivierung erfolgte bei 33°C und 80% Luftfeuchte.

Nach 48 Stunden wurde die OD bei einer Meßwellenlänge von 660 nm mit dem Biomek 1000 (Beckmann Instruments GmbH, München) ermittelt. Die Konzentration gebildeten Threonins wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 2 ist das Ergebnis des Versuchs dargestellt.

**Tabelle 2**

| Stamm | OD(660) | L-Threonin g/l |
|---|---|---|
| DSM5399/pRM17 | 13,1 | 0,61 |
| DSM5399 | 13,9 | 0,43 |

## Patentansprüche

1. Verfahren zur Herstellung von L-Aminosäuren durch Fermentation coryneformer Bakterien,
**dadurch gekennzeichnet,**
daß man Bakterien einsetzt, in denen man die für die Malat:Chinon Oxidoreduktase codierenden Nucleotidsequenzen verstärkt, insbesondere überexprimentiert.

2. Verfahren gemäß Anspruch 1,
**dadurch gekennzeichnet,**
daß man Bakterien einsetzt, in denen man zusätzlich weitere Gene des Biosyntheseweges der gewünschten L-Aminosäure verstärkt.

3. Verfahren gemäß Anspruch 1,
**dadurch gekennzeichnet,**
daß man Bakterien einsetzt, in denen die Stoffwechselwege zumindest teilweise ausgeschaltet sind, die die Bildung der gewünschten L-Aminosäure verringern.

4. Verfahren gemäß einem oder mehreren der vorhergehenden Ansprüche,
**dadurch gekennzeichnet,**
daß man einen mit einem Plasmidvektor transformierten Stamm einsetzt und der Plasmidvektor die für die Malat:Chinon Oxidoreduktase codierende Nucleotidsequenz trägt.

5. Verfahren gemäß Anspruch 4,
**dadurch gekennzeichnet,**
daß man mit dem Plasmidvektor pRM17 hinterlegt in Corynebacterium glutamicum, unter der Nummer DSM12711, transformierte Bakterien einsetzt.

6. Verfahren gemäß einem oder mehreren der vorhergehenden Ansprüche,
**dadurch gekennzeichnet,**
daß man coryneforme Bakterien verwendet, die L-Asparaginsäure, L-Asparagin, L-Homoserin, L-Threonin, L-Isoleucin oder L-Methionin herstellen.

7. Verfahren gemäß einem oder mehreren der Ansprüche 1 bis 5,
**dadurch gekennzeichnet,**
daß man coryneforme Bakterien verwendet, die L-Lysin herstellen.

8. Verfahren gemäß Anspruch 7,
**dadurch gekennzeichnet,**
daß gleichzeitig das für die Dihydrodipicolinat-Synthase kodierende dapA-Gen überexprimiert wird.

9. Verfahren gemäß Anspruch 7,
**dadurch gekennzeichnet,**
daß gleichzeitig ein S-(2-Aminoethyl)-Cystein-Resistenz vermittelndes DNA-Fragment amplifiziert wird.

10. Verfahren zur fermentativen Herstellung von L-Aminosäuren gemäß einem oder mehreren der vorhergehenden Ansprüche,
**dadurch gekennzeichnet,**
daß man folgende Schritte durchführt:
a) Fermentation der die gewünschten L-Aminosäure produzierenden Bakterien, in denen zumindest das Malat:Chinon Oxidoreduktase -Gen verstärkt wird.
b) Anreicherung der L-Aminosäure im Medium oder in den Zellen der Bakterien und
c) Isolieren der produzierten L-Aminosäure.
